Europäisches Patentamt

European Patent Office

Office européen des brevets

(11) Publication number: **0 420 635 A2**

# EUROPEAN PATENT APPLICATION

(21) Application number: 90310572.4

(22) Date of filing: 27.09.90

(51) Int. Cl.5: **C12Q 1/68**, //C12N15/30

(30) Priority: 28.09.89 GB 8921937

(43) Date of publication of application:
**03.04.91 Bulletin 91/14**

(84) Designated Contracting States:
**ES FR GB GR IT**

(71) Applicant: **Miles, Michael Alexander**
**46 The Mall**
**Southgate, London N14 6LN(GB)**

Applicant: **Howard, Michael Keith**
**13 Princes Road**
**Penge, London SE20(GB)**

Applicant: **Kelly, John Morrison**
**1 Rivington Crescent**
**Mill Hill, London NW7 2LE(GB)**

(72) Inventor: **Miles, Michael Alexander**
**46 The Mall**
**Southgate, London N14 6LN(GB)**
Inventor: **Howard, Michael Keith**
**13 Princes Road**
**Penge, London SE20(GB)**
Inventor: **Kelly, John Morrison**
**1 Rivington Crescent**
**Mill Hill, London NW7 2LE(GB)**

(74) Representative: **Perry, Robert Edward et al**
**GILL JENNINGS & EVERY 53-64 Chancery Lane**
**London WC2A 1HN(GB)**

(54) Diagnostic reagents for leishmaniasis.

(57) A sensitive repetitive DNA probe is specific to the Leishmania donovani complex and can be used as an epidemilogical and diagnostic reagent.

EP 0 420 635 A2

## DIAGNOSTIC REAGENTS FOR LEISHMANIASIS

### Field of the Invention

This invention relates to the detection of organisms of the Leishmania donovani species complex using DNA probes produced from recombinant bacteriophages which express Leishmania donovani genes.

### Background to the Invention

Leishmania is a genus of parasitic protozoa. In order to complete their life cycles, the organisms require an invertebrate sand fly host, in which the promastigote form occurs, and a vertebrate host (e.g. man) in which the intracellular amastigote form occurs.

Leishmaniae cause various forms of cutaneous, mucocutaneous and visceral leishmaniasis in man. Cutaneous leishmaniasis is found (worldwide) in the tropics and subtropics and is caused by several Leishmania species such as L. tropica , L. major , L. aethiopica and L. mexicana . The agent of South American mucocutaneous leishmaniasis is L. braziliensis which causes ulcerative lesions around the mouth, nose and pharynx.

Human visceral leishmaniasis or kala-azar is the most life-threatening of the three principal clinical forms of leishmaniasis. Visceral leishmaniasis is also found over large areas of the tropics and subtropics, especially in India, Africa, South America and the Mediterranean region. Investigations of visceral leishmaniasis are limited by the lack of good epidemiological data relating to the prevalence of L. donovani in reservoir hosts and insect vectors.

Specific articles of relevance, some in the prior art, are given in the list of references, below.

### Summary of the Invention

L. donovani cDNA clones have been isolated in an attempt to gain more understanding of the processes involved in controlling the development of infectivity in the promastigote stage of the parasite. One such clone (designated herein as Lmet2) encodes an untranslated region of a mRNA expressed in the promastigote stages of L. donovani . The clone represents a DNA sequence present only within parasites of the L. donovani complex.

According to the present invention, a genomic DNA probe is specific to the L. donovani complex. Such a probe is extremely sensitive, and detects L. donovani irrespective of geographical origin. The probe can reliably detect small numbers of organisms including promastigotes in sand flies and amastigotes in mammalian hosts.

### Description of the Invention

SEQ ID No. 1 shows the nucleotide sequence of the Lmet 2 cDNA, sense strand as determined by northern blots with single-stranded DNA probes. The sequence consists predominantly of four imperfect repeats (58-60 bp).

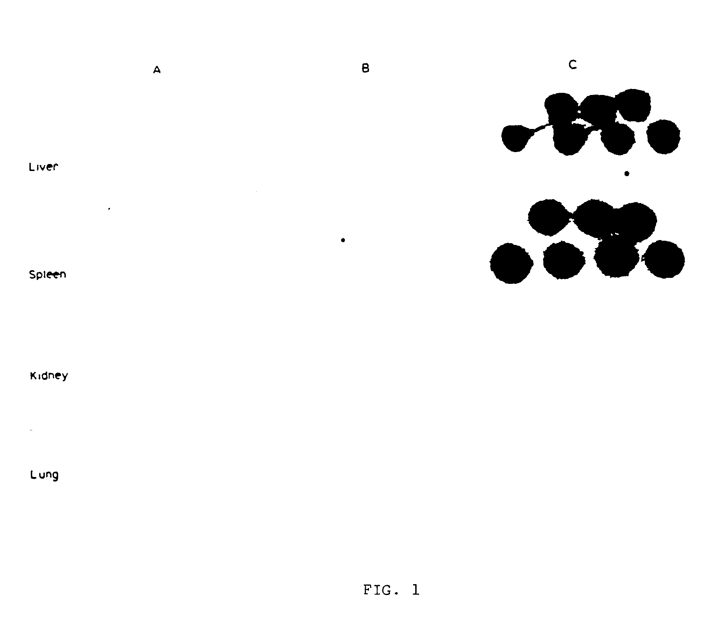

Fig. 1 shows the detection of L. donovani amastigotes within hamster tissues. Impression smears made from the liver, spleen, kidney, and lung of (A) an uninfected hamster; (B) a hamster with a low level of L. donovani infection (splenic L.D.U. value = 0.04 [28]); (C) a hamster with a high level of L. donovani infection (splenic L.D.U. value = 5.97). Seven impressions with each organ were made on nylon membranes, stringency 0.25 x SSC, 65° C, autoradiography 2 days.

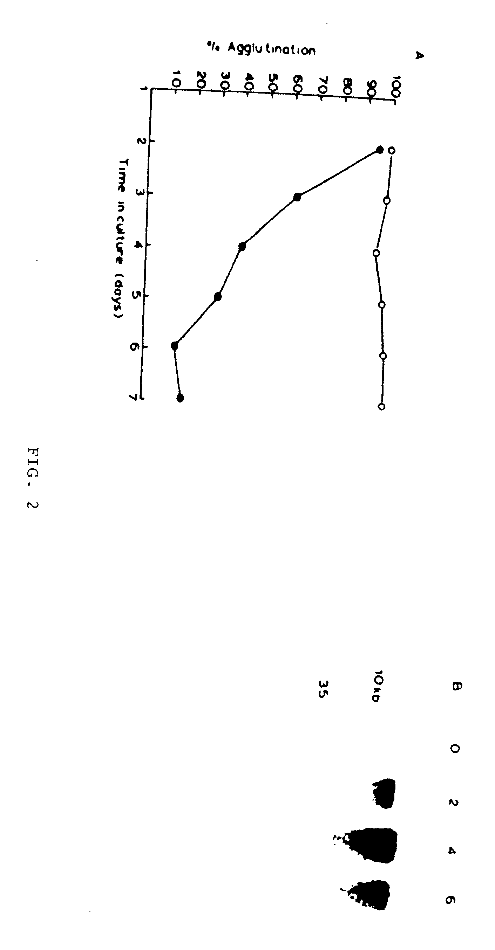

Fig. 2 shows the expression of Lmet 2 RNA during the development of Leishmania donovani metacyclic promastigotes in vitro: (A) change in lectin-mediated (125 $\mu$g/$\mu$l) agglutination of promastigotes, *, PNA, /o/l, Con A; (B) total RNA (20 $\mu$g) isolated from parasites on day 0 (amastigotes), 2, 4, and 6 of culture in transformation medium was northern blotted and hybridized with the Lmet 2 probe, stringency 0.25 x SSC, 60° C, autoradiography overnight.

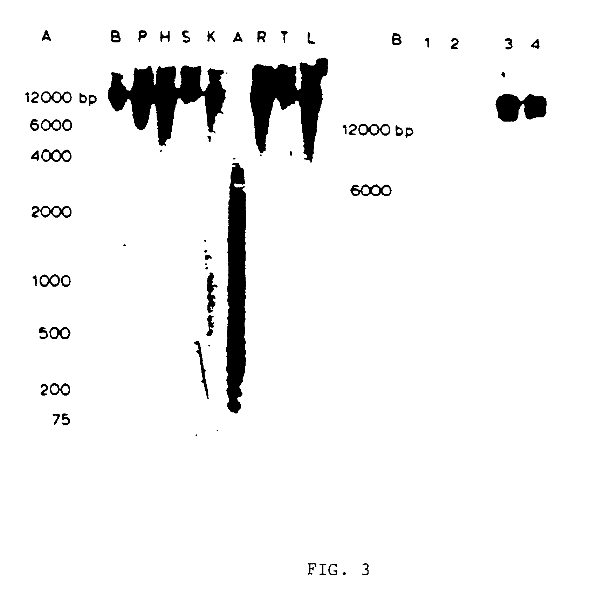

Fig. 3 shows the genomic organization of Lmet 2: (A) restriction digests of total Leishmania donovani (HU3) DNA, B, Bam H1; P, Pst I; H, Hind III; S, Sst I; K, Kpn I; A, Acc I; R, Rsa I; T, Sst II; L, Alu I; stringency 0.25 x SSC, 60° C, autoradiography 4.5 h: (B) Restriction digests with Sal I of (1) L.

braziliensis braziliensis (LTB300), (2) L. major (JISH252), (3) L. d. chagasi (PP75), (4) L. donovani s.l. (HU3), stringency 2 x SSC, 50°C, autoradiography 4.5 h.

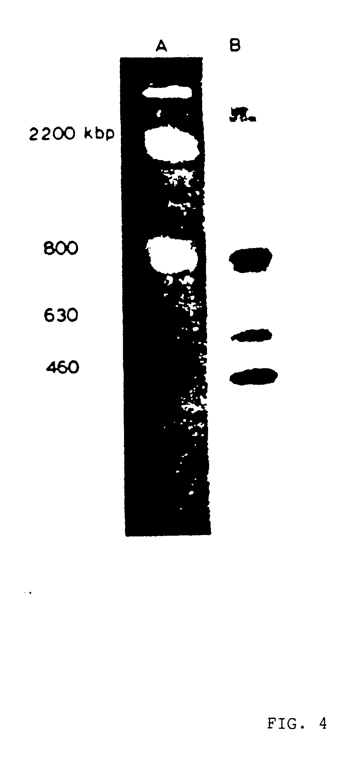

Fig. 4 shows the chromosomal location of Lmet 2. Leishmania donovani chromosomes separated on a CHEFE gel. (A) Ethidium bromide-strained; (B) Southern blotted and hybridized with Lmet 2, stringency 0.25 x SSC, 60°C, autoradiography 40 min.

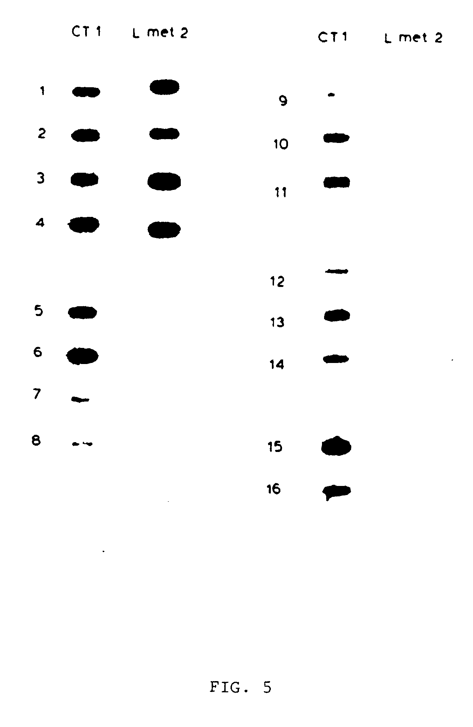

Fig. 5 shows the species-specificity of the Lmet 2 sequence. $10^5$ cells of 16 species of sub-species of Leishmania (WHO reference strains, Table 1) were hybridized with either hsp 70 (CT1) or Lmet 2 probes, stringency 0.25 x SSC, 55°C, autoradiography; overnight (CT1), or 3 h (Lmet 2) (1) L. donovani donovani (2) L. donovani s.l. , (3) L. d. infantum , (4) L. d. chagasi , (5) L. major , (6) L. arabica , (7) L. tropica , (8) L. aethiopica , (9) L. mexicana mexicana , (10) L. m. amazonensis , (11) L. Pifanoi , (12) L. braziliensis braziliensis , (13) L. b. guyanensis , (14) L. b. panamensis , (15) L. hertigi hertigi , (16) L. gerbilli . hsp 70 is a multigene family with a variable copy number in different Leishmania species [27].

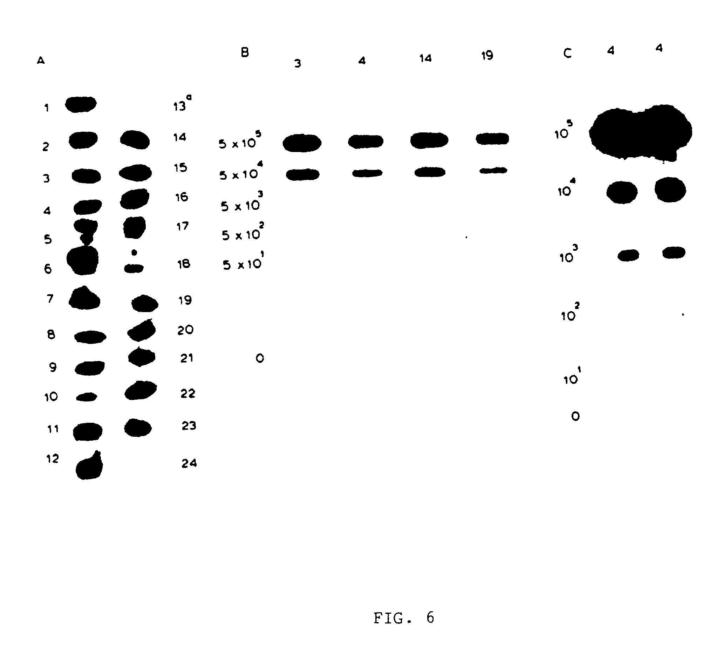

Fig. 6 shows the sensitivity of the Lmet2 probe with different Leishmania donovani strains. (A) recognition of 22 strains of L. donovani from 12 countries, India (1-3), Ethiopia (4-5), Sudan (6-7), Kenya (8-10), Lebanon (11), Italy (12), Tunisia (14), France (15), China (16-18), Brazil (19,20), Panama (21), Iraq (22,23); isolates are (Table 1): Nandi (1), Patna (2), DD8 (3), HU3 (4), Addis 151 (5), Khartoum (6), IS (7), Neal R1 (8), MRC 74 (9), MRC L57 (10), Salti 1 (11), Lara (12), IPTI (14), Pharoah (15), Wang Jei (16), Racoon dog (17), Peking (18), PP75 (19), C0910 (20), WR341 (21), BUMM 3 (22), Sukkar2 (23), LTB300 (L. b. braziliensis ) (24), stringency 0.25 x SSC, 60°C, autoradiography, 4 h. The strain 13, designated as L. d. infantum LEM75, has been shown to be L. major by isoenzyme analysis. (B) and (C) Titration of L. donovani sub-species reference strains (Table 1) and detection by the Lmet 2 probe, stringency 0.25 x SSC, 60°C, autoradiography, 4 h (B) or 2 days (C).

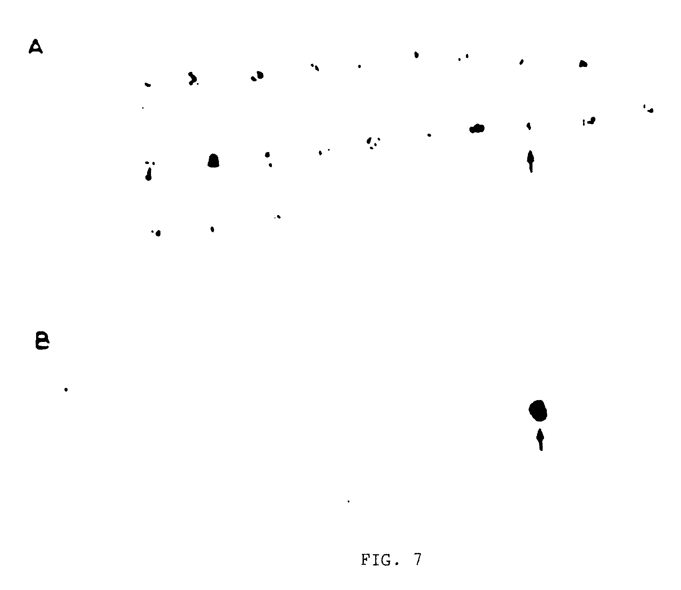

Fig. 7 shows an example of the detection of L. donovani promastigotes in Lutzomyia longipalpis using the Lmet 2 probe. (A) squashes of 23 Lutzomyia longipalpis fed on a L. donovani -infected golden hamster. (B) autoradiograph of A after hybridisation with the Lmet 2 probe. The only fly in which flagellates were seen microscopically is arrowed in A: the signal above the same fly is arrowed in B. Stringency, 0.25 x SSC, 60°C, autoradiography 2 days.

## Description of the Invention

cDNA libraries were constructed in the bacteriophages lamdba gt10 and lambda gt11 using RNA isolated from infective promastigotes of L. donovani . The lambda gt10 library was screened differentially with radio-labelled cDNA from non-infective and infective promastigotes. Plaques hybridizing only to cDNA from infective promastigotes were rescreened until single homogeneous clones were obtained, after which the clones were stored as phage suspensions.

Recombinant clone Lmet2 was identified as specific for organisms of the L. donovani complex by probing DNA and RNA isolated from different Leishmania species and immobilised on nylon membranes. Lmet2 hybridized to DNA from (twenty-two) isolates of L. donovani representing parasites from each of the areas endemic for visceral leishmaniasis but not to DNA from other Leishmania species or Trypanosoma cruzi or Plasmodium falciparum . The Lmet2 cDNA clone was subsequently used to examine the genomic structure, chromosomal location, and stage-specific expression of the gene. The clone contained a 238bp DNA insert corresponding to part of an untranslated region of a mRNA present only in promastigotes. M13 sequencing of the 238bp cDNA insert revealed a nucleotide repeat unit of approximately sixty bp.

On northern blots, using total cellular RNA, the cDNA hybridized to a 12kDa RNA transcript present in promastigotes. Data from Southern analysis of genomic DNA indicated that the gene was present either as a tandem repeat array or in a region of the chromosome(s) deficient in recognition sites for common restriction endonucleases. Contour homogeneous electric field electrophoresis [CHEFE] indicated that the gene was present on at least six chromosomes.

The Lmet2 cDNA has been used to probe squashes of sandflies blood-fed 5 days previously on hamsters infected with L. donovani . The radioactive probe produced a signal only above those flies shown by dissection and microscopical examination of the sandfly gut contents to contain flagellates. In addition, the probe has been used to identify L. donovani promastigotes within the guts of wild-caught Ethiopian sandflies (Phlebotomus martini ). Titration of L. donovani DNA on a slot-blot manifold indicated that the 32p-labelled Lmet2 probe was capable of detecting fewer than 100 parasites.

The Lmet2 probe has also been used to identify L. donovani in samples from patients suspected of suffering from visceral leishmaniasis, either with or without associated HIV infection, or, following treatment, to evaluate the success of chemotherapy. Samples involved have been bone marrow, primary cultures of bone marrow, spleen, liver, skin and blood. In some cases, diagnosis has been confirmed earlier than by conventional techniques, or has revealed residual or recrudescent infections, and demonstrated cutaneous lesions due to L. donovani .

The following illustrates how the novel products may be prepared:

1. a) Bulk preparation of recombinant phage DNA;

b) Bulk enzymic amplification of Lmet2 sequences or portions thereof;

c) Subcloning the Lmet2 cDNA, or a portion thereof, into a plasmid vector to facilitate bulk production of recombinant DNA; or

d) Bulk preparation of a synthetic oligonucleotide based on DNA sequence data.

2. Production of specific probes carrying labels such as radioactivity, enzymes, biotin, chemiluminescence, replication signals etc., for the identification of parasite DNA by Southern blot analysis or modifications thereof, by DNA or RNA slot blots, by in situ hybridization using isolated parasites or biopsy material or by squash blots and dot blots of sand fly material.

3. Production of oligonucleotide primers suitable for amplification of parasite DNA in cultured material or insect vectors using a DNA polymerase.

4. Subcloning of any sequences of the Lmet2 gene into any expression vector such a bacteriophage lambda gt11, purification of corresponding fusion proteins or fragments thereof and production of reactive antibodies to act as probes for the detection of antigens encoded by the Lmet2 gene.

Example

The following illustrates the preparation and properties of Lmet2. The same text can be found in the "Materials and Methods" and "Results" sections of the article (in press) in Molecular and Biochemical Parasitology (1991) entitled "A sensitive repetitive DNA probe that is specific to the Leishmania donovani complex and its use as an epidemiological and diagnostic reagent". The authors are M. Keith Howard, John M. Kelly, Richard P. Lane and Michael A. Miles. The article was received 21 May 1990 and accepted 25 July 1990. The relevant references are given below, with the same numbering as in the article. However, the article's Fig. 1 is now SEQ ID No. 1, and Fig. 8 is here Fig. 1.

Parasites . A list of the Leishmania strains used is given in Table 1. Promastigotes were retrieved from cryopreserved material and maintained, at 23°C, in supplemented Grace's insect medium as described [13,17]. L. donovani HU3 strain amastigotes were maintained by bi-monthly passage in golden hamsters and harvested from hamster spleen 8 weeks post-inoculation [18]. Metacyclic promastigotes of the HU3 strain were generated in vitro using Grace's transformation medium developed previously [17]. Evaluation of the proportion of metacyclic promastigotes in cultures was primarily by lectin agglutination with peanut agglutinin [PNA] [17].

Preparation of parasite RNA and DNA . Total RNA was prepared by guanidinium thiocyanate lysis of cells and centrifugation of the lysate through a 5.7M caesium chloride gradient [19]; poly(A)$^+$ RNA was purified from total RNA by oligo(dT) affinity purification [19]. For DNA preparations, $10^9$ promastigotes were washed (x2) by centrifugation in sterile phosphate-buffered saline [PBS] and then lysed in 50mM EDTA/1% SDS/50mM NaCl/50mM Tris-HCl pH 8.0 containing 100 μg/ml of proteinase K, and incubated at 37°C for 3-24 h. After phenol/chloroform extraction (x3) the DNA was ethanol-precipitated with ammonium acetate [19] and stored at +4°C in 10mM Tris-HCl/1mM EDTA, pH 7.4 [TE].

Isolation of cDNA clones . The complementary DNA (cDNA) library was constructed in λgt10 by the RNase H method [20] using 2 μg of poly(A)$^+$ RNA prepared from cultured HU3 strain metacyclic promastigotes, which were harvested 8 and 10 days after transformation cultures had been started [17]. Replicate lifts of $10^4$ plaque-forming units (pfu) were screened differentially with radio-labelled cDNA prepared from 1 μg poly(A)$^+$ RNA from HU3 strain log phase promastigotes or HU3 strain metacyclic promastigotes. 100 μg/ml of polyuridine was incorporated into prehybridization and hybridization solutions [19] and washes were at 50°C with 0.25 x SSC. A series of recombinants recognised specifically by the metacyclic cDNA probe was selected and rescreened to homogeneity.

Analysis and labelling of the cDNA Lmet 2. The cDNA clone that subsequently proved to be of interest as a specific DNA probe was designated Lmet 2. Phage DNA from this clone was prepared and insert DNA (238bp) was isolated after Eco R1 digestion [19]. Lmet 2 insert DNA was labelled by random priming [19]. Insert DNA was ligated into Eco R1 cut M13 mp18 (Amersham), single-stranded templates prepared and sequenced by the Sanger chain termination method using 'Sequenase' (US Biochemicals) [21,22].

Nucleic acid analysis . Standard protocols were used for northern transfers of RNA and Southern transfers of restriction digests of parasite genomic DNA [19]. For slot blots, $2.25x10^8$ parasites were washed

(x2) in PBS, suspended in 250 $\mu$l of 0.4M NaOH, heated to 80°C for 10 min, and then placed on ice for 5 min. Cell debris was removed by centrifugation (5000 x g, 5 min), and supernatants containing DNA were diluted with 0.4M NaOH to the equivalent parasite concentration in 100 $\mu$l aliquots. Samples were transferred to Hybond-N [Amersham] using a Hybrislot manifold [Gibco-BRL]. Wells were rinsed with 100 $\mu$l of 0.4M NaOH and membranes were removed and floated twice on 2 x SSC, air-dried and baked for 2 h at 80°C before prehybridization, probing and autoradiography (see Figure legends). Some slot blots (see Results, and Fig. 6A) were also performed by applying intact parasites to the nylon membrane. The cells were then processed as described below for sand fly squashes.

The preparation of parasites for the fractionation of chromosome-sized parasite DNA was as described [23]. Clamped, homogeneous electric field electrophoresis (CHEFE) [24] was performed using a BioRad CHEFE system. Electrophoresis was at 160 V and 1 s switching time for 30 min, followed by 160 V and 70 s switching time for 18 h, followed by 160 V at 150 s for 24 h.

Detection of Leishmania infections in sand flies . Female Lutzomyia longipalpis , from laboratory colonies, were fed on the shaved abdomen of golden hamsters infected 8 weeks previously with $10^7$ L. donovani HU3 strain amastigotes. Female flies that had fed were separated from unfed flies and maintained for 5-7 days on a sucrose diet. Flies were immobilised at -20°C, dissected in PBS, examined for flagellates and squashed onto nylon membranes [25]. Filters were layered successively onto Whatman 3 MM paper soaked with 10% SDS (2 min), 0.5 M NaOH/2.5 M NaCl [2 x 5 min] and 3M sodium acetate (pH 5:3 x 2 min) [26]. Chitinous parts were removed, the filter was air-dried at room temperature, (1 h) and baked at 80°C for 2 h. Filters were probed with radio-labelled Lmet 2 insert DNA, washed at a stringency of 0.25 x SSC at 50°C and autoradiographed at -70°C in the presence of enhancing screens.

Detection of Leishmania infections in hamsters . Impression smears from infected hamster liver, spleen, kidney and lung were made, both on microscope slides and nylon membranes (Hybond-N) eight weeks after infection with HU3 strain amastigotes. Smears from unifected hamsters were used as controls: smears on slides were stained with Giemsa and examined microscopically, smears on nylon membranes were processed in the same way as blots prepared from sand flies (above).

## Results

Characterisation of clone Lmet 2 . Clone Lmet 2 was isolated from a L. donovani (HU3 strain) metacyclic promastigote λgt10 cDNA library after differential screening with cDNA synthesised from mRNA of either metacyclic or log-phase promastigotes (see above). The clone was recognised specifically by metacyclic cDNA (not shown). Eco R1 digestion of Lmet 2 DNA produced an insert fragment of 238bp. Sequence analysis revealed that it was comprised predominantly of four copies of an imperfect 60bp repeat motif (SEQ ID No. 1). No significant similarity was found with sequences in the EMBL database. Given the degeneracy within the repeat sequences and lack of substantial open reading frames it is unlikely that the Lmet 2 sequence corresponds to the protein coding region of a transcript. The mRNA was identified as a 12 kb promastigote-specific transcript (Fig. 2B) which was sensitive to RNase A digestion (not shown). Increased expression of Lmet 2 RNA was correlated with development of a metacyclic phenotype (Fig. 2B). The principal criterion for production of metacyclic promastigotes in this culture system was the appearance of PNA⁻ organisms (Fig. 2A), although other criteria were also used (17). On autoradiographs of northern blots with reduced RNA concentrations two adjacent bands could be resolved (not shown).

Southern analysis of L. donovani HU3 genomic DNA cut with a range of restriction enzymes revealed a predominant single or doublet high molecular weight band (18-20 kb) which hybridised with Lmet 2 insert DNA (Fig. 3A, 3B). The absence of lower molecular bands, even when four cutter enzymes were used, suggests the presence of multiple repeated copies of the 60bp motif. The Lmet 2 insert DNA contains a single Acc I site (GTCTACT, nucleotides 217-223). Southern analysis of Acc I digested genomic DNA identified two bands of 60 and 350 bp after autoradiography (not shown). Partial digestion (Fig. 3A) produced a ladder of bands consistent with an extensive tandemly-repeated arrangement.

Hybridisation of Lmet 2 DNA to L. donovani chromosome-sized DNA transferred from CHEFE gels indicated that related sequences were distributed among at least 6 chromosomes of sizes varying from 490 kb to > 2200 kb (Fig. 4).

Species-specificity and sensitivity of the Lmet 2 sequence. Southern blot analysis of L. donovani (HU3), L. major (JISH 252) and L. braziliensis braziliensis (LTB 300) genomic DNA with the Lmet 2 insert DNA probe originally suggested that the Lmet 2 sequence might be species-specific or strain-specific (Fig. 3B). DNA was prepared from sixteen WHO reference strains, transferred to slot blots and hybridized with the Lmet 2 probe. The Lmet 2 probe hybridized strongly and with absolute specificity to L. donovani donovani ,

5

L. donovani s. l., L. donovani infantum and L. donovani chagasi (Fig. 5). By way of comparison, a 70-kDa heat-shock protein gene (hsp 70) probe (CT1) [27] isolated from the same strain of L. donovani hybridized to all WHO reference strains (Fig. 5). The Lmet 2 probe specificity held at all washing stringencies used (between 2 x SSC at 50°C and 0.25 x SSC at 65°C). The DNA slot blot shown in Fig. 5 further suggests that the signal produced by hybridization of the radio-labelled Lmet 2 probe was of approximately equal intensity across the L. donovani complex. Accordingly DNA from 22 strains of the L. donovani complex (L. d. donovani , L. donovani sensu lato , L. d. infantum and L. d. chagasi ) was probed with Lmet 2. All strains were clearly and unequivocally detected by the Lmet 2 probe, irrespective of geographical origin, which encompassed very widely dispersed endemic regions (Fig. 6).

The equivalent sensitivity of the Lmet 2 probe for organisms from different taxonomic divisions of the L. donovani complex was confirmed by slot blot titrations of parasite DNA (Fig. 6B, autoradiography for 4 h). More prolonged autoradiography demonstrated that the radiolabelled probe could readily detect DNA from fewer than 100 organisms (Fig. 6C).

Detection of L. donovani parasites in Lutzomyia longipalpis . Laboratory-bred Lutzomyia longipalpis were fed on hamsters infected eight weeks previously with amastigotes of L. donovani (HU3). Those sand flies surviving six days after feeding were dissected and examined microscopically. The "reassembled" abdomen and thorax were then squashed onto a nylon membrane (see above). The squash-blots were processed and probed blind with radio-labelled Lmet 2 insert probe (Fig. 7). A total of 47 Lu. longipalpis from two separate experiments have been dissected and examined in this way: three were seen to contain flagellates, one with a very light infection (approximately 10 organisms seen). Infections in all microscopically positive flies were detected by the Lmet 2 probe. Some sand flies were seen to contain blood meal remnants but this did not give rise to non-specific signals.

Detection of amastigotes in hamster organs . As a preliminary test of the ability of the Lmet 2 probe to detect amastigote infections in biopsy samples, touch blots, onto glass slides and nylon membrane, were made from the liver, spleen, kidney, and lung of two hamsters infected eight weeks previously with L. donovani (HU3). Examination of Giemsa stained preparations showed that hamster B (Fig. 1) had a light infection (L.D.U. index [28] values: liver, 0.005; spleen, 0.04; kidney, 0.005; lung, 0.05). The infections were clearly detected in organs with L.D.U. values of at least 0.05 (Fig. 1). No signal was seen with blots prepared in precisely the same way from an uninfected hamster (Fig. 1A).

In conclusion, Lmet 2 has potential for use in direct diagnosis of L. donovani infections. The cDNA sequence is encoded as part of a multigene family. It is arranged as multiple copies of a 60bp degenerate sequence which is present on at least six chromosomes (Fig. 4) and is expressed within a 12 kb promastigote-specific transcript (Fig. 2B). The absence of a substantial open reading frame and the inability to translate the 60bp motif as an amino acid repeat suggest that the sequence represents part of the 3'- or 5'- untranslated region of the RNA. The identities of the associated proteins have not been determined.

The species-specificity of the Lmet 2 sequence was clear-cut, with no corresponding sequences apparent in other Leishmania species even when genomic Southern blots were washed at low stringency (2 x SSC, 50°C, Fig. 3B) prior to autoradiography. Lmet 2 was specific for members of the L. donovani complex, including L. d. chagasi , supporting the inclusion of the latter in the complex and its close relationship to the Old World members of the group.

The exclusive presence of the Lmet 2 sequence in the L. donovani complex, with multiple copies in all the L. donovani strains that have been examined, suggests that it is valuable as an epidemiological and diagnostic tool. Leishmania infections cause a wide spectrum of disease. The causative agents are morphologically similar and difficult to isolate into culture medium, from vectors or mammalian tissues, due to contamination or fastidious growth requirements. Reliable identification by isoenzyme electrophoresis [29] or by the analysis of extracted kDNA [11] requires cultivation of more than 10 promastigotes. Although species-specific monoclonal antibodies can be used to identify small numbers of promastigotes or amastigotes they are often strain or stage-specific and panels of antibodies are required for accurate identification [30]. DNA probes have the advantage that they are not stage-specific and the selection of isolate-specific [31], species-specific [14,32] or subgenus-specific [12] probes is possible.

Total kDNA probes can be used to distinguish between isolates of L. braziliensis and L. mexicana and have been used to detect organisms derived from lesion biopsies and wild caught sandflies [16,33,34].

Regions of strong homology between Old World species kDNA require the selective cloning of species-specific kDNA fragments [31]. Such recombinant L. major , and L. donovani , kDNA probes have been used to detect parasites in experimentally infected sand flies, in lesion biopsies and in buffy coat cells [14,25,35]. Additional recombinant kDNA probes have been described for the identification of L. tropica [31] L. aethiopica [31] and L. donovani [36].

The high copy number of kDNA sequences theoretically presents a multiple target for DNA probes. In practice, kDNA sequence diversity frequently leads to probes of diffuse specificity and 50- to 100-fold variation in sensitivity for different isolates [3,12,14,35,36]. Genomic DNA or cDNA probes have been used to distinguish Old World Leishmania species but only following Southern blot analysis [12,13] and are therefore of little value for the rapid field identification of Leishmania infections in vectors or mammalian hosts.

The properties of the Lmet 2 probe compare favourably with those of published kDNA probes: sensitivity is in some cases 10- to 100-fold greater, no cross-hybridization has been detected with other Leishmania taxa (Fig. 5), and there is no significant variation in signal between target isolates of the L. donovani complex.

Low-level L. donovani infections in experimentally infected sand flies typical of those seen in naturally infected, field caught flies were reliably detected with this probe (Fig. 7). Indeed, preliminary work has confirmed the value of the probe as an epidemiological tool to detect infections in wild caught sand flies in both Ethiopia and in Brazil.

The sensitivity of the probe for detection of infections in sand flies suggests that it might have several other applications, such as: the detection of human cutaneous leishmaniasis due to L. donovani ; the diagnosis of cryptic, asymptomatic L. donovani infections; the early diagnosis of AIDS-associated visceral leishmaniasis; monitoring response to chemotherapy and the veterinary parasitological diagnosis of canine infections or implication of other reservoir hosts. Preliminary trials of some of these applications are in progress and successful clinical and canine diagnoses have been made. Amplification by PCR with two oligonucleotides complementary to portions of the Lmet 2 sequence yields an intense product smear on ethidium bromide stained gels, as expected for such a repeat sequence (not shown). A simplified PCR-based assay might be developed for well-equipped clinical diagnostic laboratories.

All tests to date indicate that the Lmet 2 probe is a potential universal reagent for parasitological diagnosis of L. donovani infections, and that, in a non-radioactive detection system, it would be applicable in the field and of significant clinical and epidemiological value.

The 238bp sequence of Lmet 2 is given as SEQ ID No.1. The invention also relates to functional fragments thereof, having the ability to identify L. donovani specifically, e.g. of 15 to 60 nucleotides. Such a functional fragment can be prepared and tested by known means and, if desired, produced synthetically.

TABLE 1: <u>Leishmania</u> strains.

| <u>International Code</u> | <u>Species/subspecies</u> | <u>Country of origin</u> |
|---|---|---|
| MHOM/IN/80/DD8 | <u>L. donovani donovani</u> | India |
| MHOM/IN/82/Patna2 | <u>L. d. donovani</u> | India |
| MHOM/IN/82/Nandi3 | <u>L. d. donovani</u> | India |
| MHOM/ET/67/HU3 | <u>L. donovani s.l.</u> | Ethiopia |
| MHOM/ET/84/Addis151 | <u>L. donovani s.l.</u> | Ethiopia |
| MHOM/SD/00/Khartoum | <u>L. donovani s.l.</u> | Sudan |
| MHOM/SD/68/1S | <u>L. donovani s.l.</u> | Sudan |
| MHOM/KE/00/NealR1 | <u>L. donovani s.l.</u> | Kenya |
| MHOM/KE/73/MRC74 | <u>L. donovani s.l.</u> | Kenya |
| IMAR/KE/62/MRCL57 | <u>L. donovani s.l.</u> | Kenya |
| MHOM/IQ/77/BUMM3 | <u>L. donovani s.l.</u> | Iraq |
| MCAN/IQ/81/Sukkar2 | <u>L. donovani s.l.</u> | Iraq |
| MHOM/TN/80/IPT1 | <u>L. d. infantum</u> | Tunisia |
| MCAN/FR/82/Pharoah | <u>L. d. infantum</u> | France |
| MCAN/IT/76/Lara | <u>L. d. infantum</u> | Italy |
| MHOM/LB/84/Salti1 | <u>L. d. infantum</u> | Lebanon |
| MHOM/CN/00/Wang Jei1 | <u>L. d. infantum</u> | China |
| MNYT/CN/80/RacoonDog | <u>L. d. infantum</u> | China |
| MHOM/CN/54/Peking | <u>L. d. infantum</u> | China |
| MHOM/BR/74/PP75(M2682) | <u>L. d. chagasi</u> | Brazil |
| MCAN/BR/84/CO910 | <u>L. d. chagasi</u> | Brazil |
| MHOM/PA/80/WR341 | <u>L. d. chagasi</u> | Panama |
| MHOM/SU/73/5ASKH | <u>L. major</u> | Sudan |
| MHOM/SA/84/JISH252 | <u>L. major</u> | Saudi Arabia |
| MPSM/SA/83/JISH220 | <u>L. arabica</u> | Saudi Arabia |

| MHOM/SU74/K27 | L. tropica | Sudan |
|---|---|---|
| MHOM/ET/72/L100 | L. aethiopica | Ethiopia |
| MRHO/CN/60/Gerbilli | L. gerbilli | China |
| MHOM/BZ/82/BEL21 | L. mexicana mexicana | Belize |
| MHOM/BR/67/PH8 | L. m. amazonensis | Brazil |
| MHOM/VE/57/LL1 | L. m. pifanoi | Venezuela |
| MHOM/BR/76/LTB300 | L. braziliensis braziliensis | Brazil |
| MHOM/BR/75/M4147 | L. b. guyanensis | Brazil |
| MHOM/PA/71/LS94 | L. b. panamensis | Panama |
| MCOE/PA/65/C8 | L. hertigi hertigi | Panama |

References

1. World Health Organization (1984) The Leishmaniases. WHO Technical Report Series 701, World Health Organization, Geneva, Switzerland.

2. Rees, P.H. and Kager, P.A. (1987) Visceral leishmaniasis and post-kala-azar dermal leishmaniasis. In: The Leishmaniases in Biology and Medicine, Vol 2 (Peters, W. and Killick-Kendrick, R, eds.), pp. 583-615, Academic Press, London.

3. Carvalho, E.M., Sampaio, D., Bacelar, O., Barral, A., Badaro, R. and Barral-Netto, M. (1988) Natural history of the immune response on visceral leishmaniasis. Mem. Inst. Oswaldo Cruz, Rio de Janeiro (Suppl. I) 83, (1M-14) 118.

4. Sacks, D.L., Lal, S.L., Shrivastava, SN., Blackwell, J.M. and Neva, F.A. (1987) An analysis of T cell responsiveness in Indian kala-azar. J. Immunol. 138, 908-913.

5. Alvar, J., Blazquez, J. and Najera, R. (1989) Association of visceral leishmaniasis and human immunodeficiency virus infections. J. Infect. Dis. 160, 560-561.

6. Peters, W. and Killick-Kendrick, R. (eds.) (1987) The Leishmaniases in Biology and Medicine, Academic Press, London.

7. Lainson R., Shaw, J.J., and Lins, Z.Z. (1969) Leishmaniasis in Brazil. IV. The fox, Cerdocyon thous (L) as a reservoir of Leishmania donovani in Pará State, Brazil. Trans. R. Soc. Trop. Med. Hyg. 76, 830-832.

8. Killick-Kendrick, R. (1979) Biology of Leishmania in phlebotomine sandflies. In: Biology of the Kinetoplastida (Lumsden, W.H.R and Evans, D.A., eds.), Vol. 2, pp. 395-460, Academic Press, New York.

9. Jaffe, J.L. and McMahon-Pratt, D. (1987) Serodiagnostic assay for visceral leishmaniasis employing monoclonal antibodies Trans. R. Soc. Trop. Med. Hyg. 81, 587-594.

10. Miles, M.A. (1985) Biochemical identification of the Leishmanias . PAHO Bulletin 19, 343-353.

11. Barker, D.C. (1989) Molecular approaches to DNA diagnosis. Parasitology 99 (Suppl S125-S146).

12. van Eys, G.J.J., Schoone, G.J., Ligthart, G.S., Alvar, J., Evans, DA and Terpstra, WJ (1989) Identification of "Old World" Leishmania by DNA recombinant probes. Mol. Biochem. Parasitol. 34, 53-62.

13. Bishop, R.P. and Akinsehinwa, F. (1989) Characterization of Leishmania donovani stocks by genomic DNA heterogeneity and molecular karyotype. Trans. R. Soc. Trop. Med. Hyg. 83, 629-634.

14. Smith, D.F., Searle, S., Ready, P.D., Gramiccia, M. and Ben-Ismail, R. (1989) A kinetoplast DNA probe diagnostic for Leishmania major : sequence homologies between regions of Leishmania minicircles. Mol. Biochem. Parasitol. 37, 213-224.

15. Sturn, N.R., Degrave, W., Morel, C. and Simpson, L. (1988) Sensitive detection and schizodeme classification of Tryoanosoma cruzi cells by amplification of kinetoplast minicircle DNA sequences: use in diagnosis of Chagas' disease. Mol. Biochem. Parasitol. 33, 205-214.

16. Wirth, D.F. and Pratt, D.M. (1982) Rapid identification of Leishmania species by specific hybridization of kinetoplast DNA in cutaneous lesions. Proc. Natl. Acad. Sci. USA 79, 6999-7003.

17. Howard, M.K., Sayers, G. and Miles, M.A. (1987) Leishmania donovani metacyclic promastigotes: transformation in vitro, lectin agglutination, complement resistance, and infectivity. Exp. Parasitol. 64, 147-156.

18. Channon, J.Y., Roberts, M.B. and Blackwell, J.M. (1984) A study of the differential respiratory burst activity elicited by promastigotes and amastigotes of Leishmania donovani in murine resident peritoneal macrophages. Immunol. 53, 345-355.

19. Maniatis, T., Frisch, E.M. and Sambrook, J. (1982) Molecular cloning: a laboratory manual. Cold Spring Harbor Laboratory, Cold Spring Harbor, NY, USA.

20. Gubler, V. and Hoffman, B.J. (1983) A simple method for generating cDNA libraries. Gene 25, 263-269.

21. Messing, J. (1983) New M13 vectors for cloning. Methods Enzymol. 101, 20-78.

22. Sanger, F., Nicklen, S. and Coulson, A.R. (1977) DNA sequencing with chain-terminating inhibitors. Proc. Natl. Acad. Sci. USA 74, 5463-5467.

23. Bishop, R.P. and Miles, M.A. (1987) Chromosome size polymorphisms of Leishmania donovani . Mol. Biochem. Parasitol. 24, 263-272.

24. Chu, G., Vollrath, D. and Davis, R.W. (1986) Separation of large DNA molecules by contour-clamped homogenous electric fields. Science 234, 1582-1585.

25. Ready, P.D., Smith, D.F. and Killick-Kendrick, R. (1988) DNA hybridizations on squash-blotted sand flies to identify both Phlebotomus papatasi and infecting Leishmania major . Med. Vat. Entomol. 2, 109-116.

26. Anxolabehere, D., Nouaud, D., Periquet, G. and Tchen, P. (1985) P-element distribution in Eurasian populations of Drosophila melanogaster : a genetic and molecular analysis. Proc. Natl. Acad. Sci. USA 82, 5418-5422.

27. MacFarlane, J., Blaxter, M.L., Bishop, R.P., Miles, M.A. and Kelly, J.M. (1990) Identification and characterisation of a Leishmania donovani antigen belonging to the 70kDa heat shock protein family Eur. J. Biochem. in press.

28. Bradley, D.J. and Kirkley, J. (1977) Regulation of Leishmania populations within the host. The variable course of Leishmania donovani infections in mice. Clin. Exp. Immunol. 30, 119-129.

29. Evans, D. (ed) (1989) Handbook on isolation, characterization and cryopreservation of Leishmania . UNDP/World Bank/WHO Special Programme for Research and Training in Tropical Diseases (TDR), Geneva, Switzerland

30. Grimaldi, G., Tesh, R.B. and McMahon-Pratt, D. (1989) A review of the geographic distribution and epidemiology of leishmaniasis in the New World. Am. J. Trop. Med. Hyg. 41, 687-725.

31. Kennedy, W.P.K. (1984) Novel identification of differences in the kinetoplast DNA of Leishmania isolates by recombinant DNA techniques and in situ hybridization. Mol. Biochem. Parasitol. 12, 313-325.

32. Lopes, U.G. and Wirth, D.F. (1986) Identification of visceral Leishmania species with cloned sequences of kinetoplast DNA. Mol. Biochem. Parasitol. 20, 77-84.

33. Rogers, W.O., Burnheim, P.F. and Wirth, D.F. (1988) Detection of Leishmania within sand flies by kinetoplast DNA. hybridization. Am. J. Trop. Med. Hyg. 39, 434-439.

34. Barker, D.C. and Butcher, J. (1983) The use of DNA probes in the identification of leishmaniasis: discrimination between isolates of the Leishmania mexicana and L. braziliensis complexes. Trans. R. Soc. Trop. Med. Hyg. 77, 285-297.

35. Jackson, P.R., Lawrie, J.M., Stiteler, J.M., Hawkins, D.W., Wolhieter, J.A. and Rowton, E.D. (1986) Detection and characterization of Leishmania species and strains from mammals and vectors by hybridization and restriction endonuclease digestion of kinetoplast DNA. Vet. Parasitol. 20, 195-215.

36. Lawrie, J.M., Jackson, P.R., Stiteler, J.M. and Hockmeyer, W.T. (1985) Identification of pathogenic Leishmania promastigotes by DNA: DNA hybridization with kinetoplast DNA cloned into E. coli plasmids. Am. J. Trop. Med. Hyg. 32, 347-355.

## Sequence Listing

SEQ ID No.1

SEQUENCE TYPE:  nucleotide

MOLECULE TYPE: cDNA

SEQUENCE LENGTH: 238 bp

ORIGINAL SOURCE ORGANISM: <u>L</u>. <u>donovani</u> promastigote

IMMEDIATE EXPERIMENTAL SOURCE: λgt10 cDNA library

PROPERTIES:  specific for organisms of the <u>L</u>. <u>donovani</u> complex

```
GTGTGTTCGG GTGGTGGTGG GCCAACTTTT CTTCCTTTGT ATCTACTGAT GGGCCATGTG   60

TGTGTTCGGG TGGTGGTGGG CCAACTTTTC TTCTTTGTAT CTACTGATGG GCCATGTGTG  120

TGTGCGGGTG GTGGTGGGCC AACTTTTCTT CTTTGTATCT ACTGATGGGC CATGTGTGTG  180

TGTGAGTGGT GGTGGGCCAA CTTTTCTTTG CTTTGTGTCT ACTGATGGGC CATGTGTG    238
```

**Claims**

1. A probe specific for organisms of the Leishmania donovani complex.
2. cDNA including the nucleotide sequence defined herein as SEQ ID No.1, or a fragment thereof comprising one or more of the imperfect 58-60 bp repeat sequences, or a fragment or substantial structural analogue thereof having substantially the same function.
3. A probe according to claim 1, for the diagnosis of visceral leishmaniasis.
4. A cDNA according to claim 2, for the diagnosis of visceral leishmaniasis.

A                    B                    C

Liver

Spleen

Kidney

Lung

FIG. 1

EP 0 420 635 A2

FIG. 2

13

FIG. 3

FIG. 4

FIG. 5

FIG. 6

A

B

FIG. 7